# EUROPÄISCHE PATENTANMELDUNG

(11) **EP 4 098 232 A1**
(43) Veröffentlichungstag der Anmeldung: **07.12.2022**
(21) Anmeldenummer: 21176791.8
(22) Anmeldetag: 31.05.2021
(51) Int. Cl.: A61F 5/453, A61F 2/00, A61F 5/48

(54) **VORRICHTUNG ZUM VERHINDERN EINES UNKONTROLLIERTEN AUSFLUSSES VON URIN**

(71) Anmelder: Krug, Thomas, 44265 Dortmund (DE); Reiter, Jens, 44139 Dortmund (DE)
(72) Erfinder: Krug, Thomas, 44265 Dortmund (DE)
(74) Vertreter: Remus, Alvaro Johannes

(57) **Zusammenfassung**

Die Erfindung betrifft eine Vorrichtung 1 zum Verhindern eines unkontrollierten Ausflusses von Urin bei männlichen Benutzern, die ein Befestigungsmittel 2 zum Befestigen der Vorrichtung 1 am Penis eines Benutzers umfasst. Das Befestigungsmittel 2 umfasst ein ringförmig bzw. elliptisch angeordnetes Band 3, das eine erste Öffnung 4 bildet, welche zur Aufnahme der Vorhaut des Penis vorgesehen ist und diese zu diesem Zweck umschließen kann. Die erste Öffnung 4 ist ausschließlich für die Aufnahme der Vorhaut des Penis vorgesehen, wobei der Durchmesser d der ersten Öffnung 4 zum Abschnüren der Vorhaut verringerbar ist. Auf diese Weise kann der Durchmesser d des Befestigungsmittels 2 stabil eingestellt werden, um die Vorhaut des Penis des Benutzers einerseits flüssigkeitsdicht und andereseits schonend abzuschnüren.

## Beschreibung

### Gegenstand der Erfindung

Die Erfindung betrifft eine Vorrichtung zum Verhindern eines unkontrollierten Ausflusses von Urin bei männlichen Benutzern, welche mindestens ein Befestigungsmittel zum Befestigen der Vorrichtung am Penis eines Benutzers umfasst, wobei das Befestigungsmittel mindestens eine erste Öffnung mit mindestens einem reversibel veränderbaren Durchmesser zur Aufnahme eines Teils des Penis umfasst, oder wobei mindestens eine erste Öffnung mit mindestens einem reversibel veränderbaren Durchmesser als Befestigungsmittel zur Aufnahme eines Teils des Penis formbar ist.

### Hintergrund der Erfindung

Harninkontinenz, im allgemeinen Sprachgebrauch zumeist als "Blasenschwäche" bezeichnet, ist das Unvermögen willkürlich, d. h. zur passenden Zeit und an einem geeigneten Ort, die Blase zu entleeren. Dieser Verlust der Fähigkeit, selbst den Ort und Zeitpunkt der Entleerung zu bestimmen, ist eine häufige Erkrankung, die etwa 50 Prozent aller über 50-jährigen Frauen betrifft. Auch wenn Frauen häufiger betroffen sind als Männer, sind solche Probleme auch bei Männern weit verbreitet, wobei die Dunkelziffer recht hoch sein dürfte, da gerade Männer ihre Probleme häufig aus Scham nicht gegenüber einem Arzt bzw. einer Äztin äußern. Das Risiko für Inkontinenz steigt mit dem Lebensalter, wobei der unwillkürliche Urinverlust unterschiedlich stark ausgeprägt sein kann. Die Ausprägung reicht von einigen Tropfen Harn bis zu permanentem Harnverlust, ohne die Fähigkeit selbst geringe Mengen Harn zu halten. Betroffene sind durch den unwillkürlichen Harnverlust im Alltag oft stark beeinträchtigt, was zu einer Minderung der Lebensqualität bis hin zur sozialen Isolation führen kann.

Selbst bei leichterer Ausprägung einer Blasenschwäche führt der unkontrollierte Abgang von Urin zu einer lästigen Geruchsbildung, Verschmutzung der Unterwäsche und einem unangenehmen Feuchtgefühl im Schritt. Zur Linderung dieser Beeinträchtigungen werden auf dem Markt derzeit ausschließlich SlipEinlagen, Windeln, spezielle Unterwäsche, Katheter oder ggf. Medikamente angeboten.

### Stand der Technik

Aus der DE 694 01 952 T2 ist beispielsweise ein externer Harnkatheter zur Linderung von Harninkontinenz bei Männern bekannt, der ein inneres Katheterelement, das unter der Vorhaut in Oberflächenkontakt mit der Eichel des Penis platziert wird, und ein äußeres Halteelement zur Befestigung des inneren Katheterelements im Gebrauchszustand umfasst. Das äußere Halteelement ist ein separates Element, das eine Auslauftülle umhüllt und in Bezug darauf axial verschiebbar ist. Der austretende Urin wird über das innere Katheterelement und die Auslauftülle, welche die Verbindung des Katheterelements mit einem Schlauch darstellt, in einen Urinsammelbeutel geleitet. Wie bei allen Katheterlösungen ist auch in diesem Fall der notwendige Urinsammelbeutel ein störendes Element, das die Bewegungsfreiheit des Benutzers deutlich einschränkt und zudem verdeckt getragen werden muss.

Die US 2019/0216657 A1 offenbart eine Inkontinenzwindel zur Verwendung durch Männer, die den Penis umschließt und mittels eines in der Windel angebrachten Riemens an dem Penis befestigt ist.

Die US 2014/0358098 A1 offenbart ferner ein Wickelsystem für inkontinente Männer, welches ein Einweg-Absorptionskissen umfasst, das den Penis gewickelt wird. Das Kissen wird durch einen ersten Klebestreifen gesichert, dann am distalen Ende zurückgefaltet und schließlich durch einen zweiten Klebestreifen, ein Gummiband oder beides an Ort und Stelle gehalten. Ein selbstklebender Peel-and-Stick-Streifen befestigt die Saugkissenbaugruppe um den Benutzer, um ein Verrutschen zu verhindern.

Wie alle Windeln, Einlagen oder entsprechende Unterwäsche haben aber auch diese beiden Windelsysteme den Nachteil, dass sie in einer Hose oder sonstiger Kleidung relativ viel Platz einnehmen und daher die Bewegungsfreiheit des Benutzers einschränken, was mit einem verschlechterten Körpergefühl einhergeht. Darüber hinaus können solche Produkte, insbesondere wenn sie bereits eine größere Menge Urin aufgenommen haben, unter der Kleidung von außen sichtbar sein. Gerade letzteres trägt häufig zu einer Minderung des Selbstbewusstsein der Benutzer dar.

### Zusammenfassung der Erfindung

Es ist Aufgabe der Erfindung, eine Vorrichtung zum Verhindern eines unkontrollierten Ausflusses von Urin bei männlichen Benutzern zur Verfügung zu stellen, welche die Nachteile der aus dem Stand der Technik bekannten Produkte vermeidet und den unkontrollierten Ausfluss von Urin bei gleichzeitig verbessertem Körpergefühl effektiv verhindert.

Erfindungsgemäß wird die Aufgabe durch eine Vorrichtung der eingangs genannten Art gelöst, bei der die erste Öffnung ausschließlich für die Aufnahme der Vorhaut des Penis vorgesehen ist, wobei der Durchmesser der ersten Öffnung zum Abschnüren der Vorhaut verringerbar ist. Mit Hilfe dieser speziellen Abbindevorrichtung kann die männliche Vorhaut effektiv und sicher abgeschnürt werden, um bei Blasenschwäche den unkontrollierten Ausfluss von Urin zu verhindern. Die abgeschnürte Vorhaut dient dabei als Barriere, die den Austritt von Urin stoppt und somit die ansonsten entstehende lästige Geruchsbildung, Verschmutzung der Unterwäsche und das unangenehme Feuchtgefühl im Schritt verhindert. Durch die Gewissheit, dass kein Urin mehr ausfließen kann, hat der Benutzer wieder ein besseres Körpergefühl, empfindet mehr Sicherheit und entwickelt dadurch wieder ein stärkeres Selbstbewusstsein. Die Erfindung beruht also auf dem neuen Prinzip, ausschließlich die männliche Vorhaut, nicht aber den Penis zum Zwecke des Zurückhaltens von Urin bei Blasenschwäche abzuschnüren. Die erfindungsgemäße Vorrichtung ist darüber hinaus wiederverwendbar, da sie den Urin nicht aufnimmt und einfach gereinigt werden kann, wenn sie beim Entfernen mit Urin in Kontakt kommt. Es handelt sich dabei nicht um ein Produkt mit medizinischer Wirkweise, die Inkontinenz oder Blasenschwäche heilen oder vermindern kann, sondern vielmehr um eine vorteilhafte Vorrichtung, die betroffenen Männern mit Blasenschwäche Sicherheit und Lebensqualität im Alltag zurückbringt, da ein unkontrollierter Urinaustritt verhindert wird. Die erfindungsgemäße Vorrichtung ist sicher und einfach zu handhaben, aufgrund der geringen Größe in der Anwendung sehr unauffällig, leicht zu reinigen sowie wiederverwendbar und somit auch nachhaltig. Die erfindungsgemäße Vorrichtung kann von jedem Mann, der eine leichte Blasenschwäche und/oder Probleme mit dem Nachtropfen beim Wasserlassen hat, ohne Weiteres angewendet werden. Darüber hinaus stellt die erfindungsgemße Vorrichtung eine unproblematische, unauffällige und sichere Alternative zur Nutzung herkömmlicher Produkten wie z.B. Slipeinlagen oder Männerwindeln dar. Sie ist ideal einsetzbar als schnelle Lösung für unterwegs, da es sich um ein kleine Vorrichtung mit einfacher Handhabung handelt.

In vorteilhafter Ausgestaltung der Erfindung ist vorgesehen, dass die erfindungsgemäße Vorrichtung ferner mindestens ein Verstellelement zum reversiblen Verändern des Durchmessers der ersten Öffnung umfasst. Mittels des Verstellelements kann der Durchmesser der ersten Öffnung schnell und zuverlässig verringert werden, um die Vorhaut effektiv und sicher abzuschnüren. Beim Entfernen der Vorrichtung kann der Durchmesser der ersten Öffnung mit Hilfe des Verstellelements leicht wieder vergrößert werden, so dass die Vorrichtung einfach wieder vom penis abgenommen werden kann. Das Verstellelement umfasst vorzugsweise mindestens eine zweite Öffnung oder mindestens eine Ausnehmung, mittels der das Verstellelement zumindest über einen Teil des Befestigungsmittels schiebbar ist. Diese vorteilhafte Ausführungsform stellt sicher, dass der Durchmesser der ersten Öffnung auf einfache Art und Weise, ohne zusätzliche Elemente bzw. Teile, verstellt und fixiert werden kann. Alternativ kann das Verstellelement in vorteilhafter Weise als separates Teil auch auf das Befestigungsmittel aufsteckbar oder an dieses anklemmbar sein. Das Verstellelement kann Teil des Befestigungsmittels oder in das Befestigungsmittel integriert sein.

In weiterer vorteilhafter Ausgestaltung der Erfindung ist vorgesehen, dass das Befestigungsmittel und/oder das Verstellelement mindestens ein Arretierungsmittel zum Einstellen und Fixieren des Durchmessers der Öffnung umfasst/umfassen. Solche Arretierungsmittel stellen das Fixieren des gewünschten Durchmessers der ersten Öffnung sicher, so dass sich der Durchmesser nicht selbstständig und unbeabsichtigt wieder vergrößern und damit den ungewollten Durchlass von Urin ermöglichen kann. Darüber hinaus ermöglicht das Vorsehen von Arretierungsmitteln ein feines Einstellen des optimalen Durchmessers, damit die Vorhaut einerseits flüssigkeitsdicht abgeschnürt und andererseits schmerzfrei und mit angenehmem Tragegefühl für den Benutzer am Penis befestigt werden kann. Das oder die Arretierungsmittel kann/können zu diesem Zweck in vorteilhafter Weise mehrere Erhebungen und/oder Ausnehmungen umfassen, welche hintereinander liegend angeordnet sind. Jede Erhebung bzw. Ausnehmung stellt dabei einen Rastpunkt dar, an dem das Arretierungsmittel das Verstellelement durch Einrasten sicher fixiert. Dabei kann/können das oder die Arretierungsmittel des Verstellelements korrespondierend zu dem oder den Arretierungsmittel(n) des Befestigungsmittels angeordnet sein.

In vorteilhafter Ausgestaltung der Erfindung ist ferner vorgesehen, dass mindestens ein Durchmesser der zweiten Öffnung oder der Ausnehmung des Verstellelements reversibel veränderbar ist. Das kann beispielsweise dadurch sichergestellt werden, dass das Verstellelement zumindest teilweise aus einem elastischen Material besteht, so dass durch entsprechendes Anpassen des Durchmessers der zweiten Öffnung ein Einrasten von Arretierungsmitteln ermöglicht wird.

In besonders vorteilhafter Ausgestaltung der Erfindung ist daher vorgesehen, dass das Befestigungsmittel und/oder das Verstellelement zumindest teilweise aus einem elastischen Kunststoffmaterial bestehen. Zumindest die Teile des Befestigungsmittels und/oder des Verstellelements, die aufgrund ihrer Funktion elastisch sein müssen und/oder die mit der Vorhaut in Kontakt kommen, sollten aus einem elastischen Material bestehen, um ihre Funktion zu gewährleisten und Druckstellen bzw. Schmerzen an der Vorhaut bzw. am Penis zu vermeiden. Aus den gleichen Gründen ist in weiterer vorteilhafter Ausgestaltung der Erfindung vorgesehen, dass das Befestigungsmittel und/oder das Verstellelement zumindest teilweise aus einem weichen Kunststoffmaterial bestehen, vorzugsweise medizinischem Silikon. Insbesondere medizinisches Silikon stellt ein nachhaltiges und biokompatibles Material dar, das aufgrund seiner Eigenschaften für die erfindungsgemäße Vorrichtung besonders gut geeignet ist. "Medizinisches Silikon" im Sinne der Erfindung umfasst eine Gruppe synthetischer Polymere (Poly(organo)siloxane), die wasserfest, aber wasserdampfdurchlässig (ermöglicht gute Hautatmung), sowie hypoallergen und somit für die menschliche Haut sehr gut verträglich sind und darüber hinaus durch ihre Wiederverwendbarkeit ein nachhaltiges Produkt darstellen.

Die Erfindung wird im Weiteren anhand der Abbildungen beispielhaft näher erläutert.

### Kurze Beschreibung der Abbildungen

**Figur 1** zeigt eine perspektivische Ansicht einer ersten Ausführungsform der erfindungsgemäßen Vorrichtung;
**Figur 2** zeigt eine perspektivische Ansicht einer zweiten Ausführungsform der erfindungsgemäßen Vorrichtung;
**Figur 3** zeigt eine perspektivische Ansicht einer dritten Ausführungsform der erfindungsgemäßen Vorrichtung;
**Figur 4** zeigt eine perspektivische Ansicht der dritten Ausführungsform der erfindungsgemäßen Vorrichtung in der Anwendung;

### Beschreibung beispielhafter und/oder bevorzugter Ausführungsformen der Erfindung

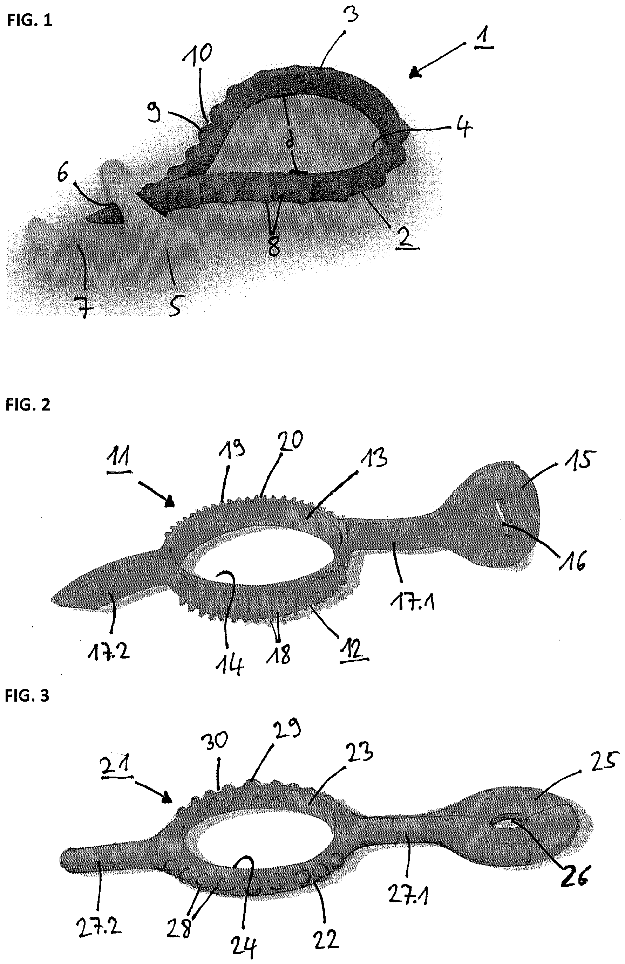

**Figur 1** zeigt eine erfindungsgemäße Vorrichtung 1, die ein Befestigungsmittel 2 zum Befestigen der Vorrichtung 1 am Penis eines Benutzers umfasst. Das Befestigungsmittel 2 umfasst ein ringförmig bzw. elliptisch angeordnetes Band 3, das eine erste Öffnung 4 bildet, welche zur Aufnahme der Vorhaut des Penis vorgesehen ist und diese zu diesem Zweck umschließen kann. Die erste Öffnung 4 ist ausschließlich für die Aufnahme der Vorhaut des Penis vorgesehen, wobei der Durchmesser d der ersten Öffnung 4 zum Abschnüren der Vorhaut verringerbar ist. Die Vorrichtung 1 weist ferner ein Verstellelement 5 auf, das zum reversiblen Verändern des Durchmessers d der ersten Öffnung 4 vorgesehen ist. Zu diesem Zweck weist das Verstellelement 5 eine zweite Öffnung 6 auf, in die eine Verlängerung 7 des Bands 3 aufgenommen ist. Das Verstellelement 5 kann in Richtung der ersten Öffnung 4 verschoben werden und somit den Durchmesser d der ersten Öffnung 4 verringern, um die Vorhaut abzuschüren. Zum Entfernen der Vorrichtung 1 vom Penis wird das Verstellelement 5 in Richtung der Verlängerung 7 zurückgeschoben, so dass sich der Durchmesser d der ersten Öffnung 4 wieder vergrößert und somit die darin zuvor eingeklemmte Vorhaut wieder frei gibt. Der Durchmesser d der ersten Öffnung 4 ist also mittels des Verstellelements 5 zur Aufnahme eines Teils des Penis (= Vorhaut) reversibel veränderbar. Das Band 3 des Befestigungsmittels 2 weist Arretierungsmittel 8 zum Einstellen und Fixieren des Durchmessers der Öffnung 4 auf. Die Arretierungsmittel 8 umfassen mehrere Erhebungen 9 bzw. Ausnehmungen 10, die auf der außen liegenden Seite des Bands 3 hintereinander liegend angeordnet sind. Das Verstellelement 5 kann im Bereich der zweiten Öffnung 6 an seiner Innenseite weitere Arretierungsmittel aufweisen (hier nicht sichtbar), die korrespondierend zu den Arretierungsmitteln 8 des Befestigungsmittels 2 angeordnet sind. Alternativ oder zusätzlich kann das Verstellelement 5 aus einem elastischen Material bestehen, so dass der Durchmesser der zweiten Öffnung 6 reversibel veränderbar ist. Durch diese vorteilhaften Ausgestaltungen des Verstellelements 5 können die Arretierungsmittel 8 des Befestigungsmittels 2 in dem Verstellelement 5 einrasten, so dass das Verstellelement 5 in einer gewünschten Position sicher und reversibel fixiert werden kann. Auf diese Weise kann der Durchmesser d des Befestigungsmittels 2 stabil eingestellt werden, um die Vorhaut des Penis des Benutzers einerseits flüssigkeitsdicht und andereseits schonend abzuschnüren.

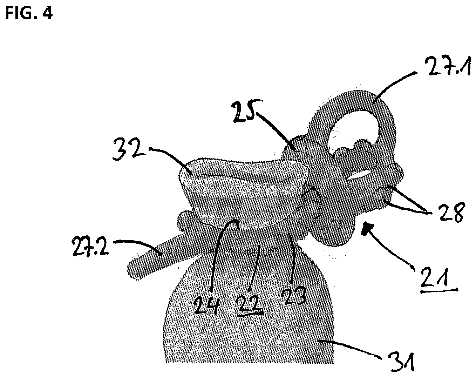

**Figur 2** zeigt eine weitere erfindungsgemäße Vorrichtung 11, die ein Befestigungsmittel 12 zum Befestigen der Vorrichtung 11 am Penis eines Benutzers umfasst. Das Befestigungsmittel 12 umfasst ein ringförmig angeordnetes Band 13, das eine erste Öffnung 14 bildet, welche zur Aufnahme der Vorhaut des Penis vorgesehen ist und diese zu diesem Zweck umschließen kann. Die erste Öffnung 14 ist ausschließlich für die Aufnahme der Vorhaut des Penis vorgesehen, wobei der Durchmesser d der ersten Öffnung 14 zum Abschnüren der Vorhaut verringerbar ist. Die Vorrichtung 11 weist ferner ein Verstellelement 15 auf, das zum reversiblen Verändern des Durchmessers d der ersten Öffnung 14 vorgesehen ist. Zu diesem Zweck weist das Verstellelement 15 eine zweite Öffnung 16 auf, in die eine erste Verlängerung 17.1 des Bands 13, die zwischen dem ringförmigen Teil des Bands 13 und dem Verstellelement 15 angeordnet ist, aufgenommen werden kann. Eine zweite Verlängerung 17.2 des Bands 13, die am gegenüberliegenden Ende des Bands 13 angeordnet ist, dient als Einfädelhilfe, mittels der das Befestigungsmittel 12 durch die zweite Öffnung 16 gezogen werden kann. Das Verstellelement 15 und die erste Verlängerung 17.1 bilden dann ein Art Schlaufe (siehe Figur 4). Das Verstellelement 15 kann dann in Richtung der ersten Öffnung 14 verschoben werden und somit den Durchmesser d der ersten Öffnung 14 verringern, um die Vorhaut abzuschüren. Zum Entfernen der Vorrichtung 11 vom Penis wird das Verstellelement 15 in Richtung der ersten Verlängerung 17.1 zurückgeschoben, so dass sich der Durchmesser d der ersten Öffnung 14 wieder vergrößert und somit die darin zuvor eingeklemmte Vorhaut wieder frei gibt. Der Durchmesser d der ersten Öffnung 14 ist also mittels des Verstellelements 15 zur Aufnahme eines Teils des Penis (= Vorhaut) reversibel veränderbar. Das Band 13 des Befestigungsmittels 12 weist Arretierungsmittel 18 zum Einstellen und Fixieren des Durchmessers der Öffnung 14 auf. Die Arretierungsmittel 18 umfassen mehrere Erhebungen 19 bzw. Ausnehmungen 20, die auf der außen liegenden Seite des Bands 13 hintereinander liegend angeordnet sind. Das Verstellelement 15 kann im Bereich der zweiten Öffnung 6 an seiner Innenseite weitere Arretierungsmittel aufweisen (hier nicht dargestellt), die korrespondierend zu den Arretierungsmitteln 18 des Befestigungsmittels 12 angeordnet sind. Alternativ oder zusätzlich kann das Verstellelement 15 aus einem elastischen Material bestehen, so dass der Durchmesser der zweiten Öffnung 16 reversibel veränderbar ist. Durch diese vorteilhaften Ausgestaltungen des Verstellelements 15 können die Arretierungsmittel 18 des Befestigungsmittels 12 in dem Verstellelement 15 einrasten, so dass das Verstellelement 15 in einer gewünschten Position sicher und reversibel fixiert werden kann. Auf diese Weise kann der Durchmesser d des Befestigungsmittels 12 stabil eingestellt werden, um die Vorhaut des Penis des Benutzers einerseits flüssigkeitsdicht und andereseits schonend abzuschnüren.

**Figur 3** zeigt eine weitere erfindungsgemäße Vorrichtung 21, die ein Befestigungsmittel 22 zum Befestigen der Vorrichtung 21 am Penis eines Benutzers umfasst. Das Befestigungsmittel 22 umfasst ein ringförmig angeordnetes Band 23, das eine erste Öffnung 24 bildet, welche zur Aufnahme der Vorhaut des Penis vorgesehen ist und diese zu diesem Zweck umschließen kann. Die erste Öffnung 24 ist ausschließlich für die Aufnahme der Vorhaut des Penis vorgesehen, wobei der Durchmesser d der ersten Öffnung 24 zum Abschnüren der Vorhaut verringerbar ist. Die Vorrichtung 21 weist ferner ein Verstellelement 25 auf, das zum reversiblen Verändern des Durchmessers d der ersten Öffnung 24 vorgesehen ist. Zu diesem Zweck weist das Verstellelement 25 eine zweite Öffnung 26 auf, in die eine erste Verlängerung 27.1 des Bands 23, die zwischen dem ringförmigen Teil des Bands 23 und dem Verstellelement 25 angeordnet ist, aufgenommen werden kann. Eine zweite Verlängerung 27.2 des Bands 23, die am gegenüberliegenden Ende des Bands 23 angeordnet ist, dient als Einfädelhilfe, mittels der das Befestigungsmittel 22 durch die zweite Öffnung 26 gezogen werden kann. Das Verstellelement 25 und die erste Verlängerung 27.1 bilden dann ein Art Schlaufe (siehe Figur 4). Das Verstellelement 25 kann dann in Richtung der ersten Öffnung 24 verschoben werden und somit den Durchmesser d der ersten Öffnung 24 verringern, um die Vorhaut abzuschüren. Zum Entfernen der Vorrichtung 21 vom Penis wird das Verstellelement 25 in Richtung der ersten Verlängerung 27.1 zurückgeschoben, so dass sich der Durchmesser d der ersten Öffnung 24 wieder vergrößert und somit die darin zuvor eingeklemmte Vorhaut wieder frei gibt. Der Durchmesser d der ersten Öffnung 24 ist also mittels des Verstellelements 25 zur Aufnahme eines Teils des Penis (= Vorhaut) reversibel veränderbar. Das Band 23 des Befestigungsmittels 22 weist Arretierungsmittel 28 zum Einstellen und Fixieren des Durchmessers der Öffnung 24 auf. Die Arretierungsmittel 28 umfassen mehrere Erhebungen 29 bzw. Ausnehmungen 30, die auf der außen liegenden Seite des Bands 23 hintereinander liegend angeordnet sind. Das Verstellelement 25 kann im Bereich der zweiten Öffnung 26 an seiner Innenseite weitere Arretierungsmittel aufweisen (hier nicht dargestellt), die korrespondierend zu den Arretierungsmitteln 28 des Befestigungsmittels 22 angeordnet sind. Alternativ oder zusätzlich kann das Verstellelement 25 aus einem elastischen Material bestehen, so dass der Durchmesser der zweiten Öffnung 26 reversibel veränderbar ist. Durch diese vorteilhaften Ausgestaltungen des Verstellelements 25 können die Arretierungsmittel 28 des Befestigungsmittels 22 in dem Verstellelement 25 einrasten, so dass das Verstellelement 25 in einer gewünschten Position sicher und reversibel fixiert werden kann. Auf diese Weise kann der Durchmesser d des Befestigungsmittels 22 stabil eingestellt werden, um die Vorhaut des Penis des Benutzers einerseits flüssigkeitsdicht und andereseits schonend abzuschnüren.

**Figur 4** zeigt die erfindungsgemäße Vorrichtung 21 gemäß Figur 3 in Anwendung am Penis 31 eines Mannes. Es wird in dieser Darstellung deutlich, dass das Befestigungsmittel 22 die Vorhaut 32 des Penis 31 umschließt und somit abschnürt. Die erste Öffnung 24 des Befestigungsmittels 22 ist ausschließlich für die Aufnahme der Vorhaut 32 vorgesehen, wobei der Durchmesser d der ersten Öffnung 24 zum Abschnüren der Vorhaut 32 verringerbar ist. Das Verstellelement 25 und die erste Verlängerung 27.1 bilden ein Art Schlaufe, wobei das Verstellelement 25 in Richtung der ersten Öffnung 24 verschoben ist und den Durchmesser d der ersten Öffnung 24 stabil verringert, um die Vorhaut 32 abzuschüren. Die Arretierungsmittel 28 des Befestigungsmittels 22 sind in dem Verstellelement 25 eingerastet, so dass das Verstellelement 25 auf der Vorhaut 32 sicher und reversibel fixiert ist, um diese flüssigkeitsdicht und gleichzeitig schonend abzuschnüren. Zum Entfernen der Vorrichtung 21 vom Penis 31 kann das Verstellelement 25 in Richtung der ersten Verlängerung 27.1 zurückgeschoben werden, so dass sich der Durchmesser d der ersten Öffnung 24 wieder vergrößert und somit die darin zuvor eingeklemmte Vorhaut 32 wieder frei gibt.

### Handhabung der erfindungsgemäßen Vorrichtung:

Zum Anbringen der Vorrichtung zieht der Benutzer die Vorhaut nach vorn (nur die Vorhaut, nicht den Penis!). Dann stülpt er die Vorrichtung über die Vorhaut und verschiebt das Verstellelement solange in Richtung des Befestigungsmittels bis sich das Band des Befestigungsmittels an die Vorhaut anschmiegt und diese so weit zusammendrückt, dass keine Flüssigkeit mehr unkontrolliert entweichen kann. Nachdem die Vorhaut so weit zugeschnürt ist, dass kein Urin mehr unkontrolliert aus dem Penis entweichen kann, sammelt sich der austretende Urin zwischen der Vorhaut und der Eichel des Penis. Beim nächsten Toilettengang kann der Benutzer dann die erfindungsgemäße Vorrichtung schnell und einfach lösen, entfernen und den gegebenenfalls angesammelten Urin über die Toilette entsorgen. Die vorzugsweise aus medizinischem Silikon hergestellte Vorrichtung kann dann einfach mit Wasser abgespült werden und ist somit leicht zu reinigen, wiederverwendbar und daher auch nachhaltig.

## Patentansprüche

1. Vorrichtung (1, 11, 21) zum Verhindern eines unkontrollierten Ausflusses von Urin bei männlichen Benutzern, welche mindestens ein Befestigungsmittel (2, 12, 22) zum Befestigen der Vorrichtung (1, 11, 21) am Penis (31) eines Benutzers umfasst, wobei das Befestigungsmittel (2, 12, 22) mindestens eine erste Öffnung (4, 14, 24) mit mindestens einem reversibel veränderbaren Durchmesser (d) zur Aufnahme eines Teils des Penis (31) umfasst, oder wobei mindestens eine erste Öffnung (4, 14, 24) mit mindestens einem reversibel veränderbaren Durchmesser (d) als Befestigungsmittel (2, 12, 22) zur Aufnahme eines Teils des Penis (31) formbar ist, **dadurch gekennzeichnet, dass** die erste Öffnung (4, 14, 24) ausschließlich für die Aufnahme der Vorhaut (32) des Penis (31) vorgesehen ist, wobei der Durchmesser (d) der ersten Öffnung (4, 14, 24) zum Abschnüren der Vorhaut (32) verringerbar ist.

2. Vorrichtung nach Anspruch 1, **dadurch gekennzeichnet, dass** diese ferner mindestens ein Verstellelement (5, 15, 25) zum reversiblen Verändern des Durchmessers (d) der ersten Öffnung (4, 14, 24) umfasst.

3. Vorrichtung nach Anspruch 2, **dadurch gekennzeichnet, dass** das Verstellelement (5, 15, 25) mindestens eine zweite Öffnung (6, 16, 26) oder mindestens eine Ausnehmung umfasst, mittels der das Verstellelement (5, 15, 25) zumindest über einen Teil des Befestigungsmittels (2, 12, 22) schiebbar ist.

4. Vorrichtung nach Anspruch 2, **dadurch gekennzeichnet, dass** das Verstellelement (5, 15, 25) auf das Befestigungsmittel (2, 12, 22) aufsteckbar oder an dieses anklemmbar ist.

5. Vorrichtung nach einem der Ansprüche 2 bis 4, **dadurch gekennzeichnet, dass** das Verstellelement (5, 15, 25) Teil des Befestigungsmittels (2, 12, 22) oder in das Befestigungsmittel (2, 12, 22) integriert ist.

6. Vorrichtung nach einem der Ansprüche 1 bis 5, **dadurch gekennzeichnet, dass** das Befestigungsmittel (2, 12, 22) und/oder das Verstellelement (5, 15, 25) mindestens ein Arretierungsmittel (8, 18, 28) zum Einstellen und Fixieren des Durchmessers (d) der Öffnung (4, 14, 24) umfasst/umfassen.

7. Vorrichtung nach Anspruch 6, **dadurch gekennzeichnet, dass** das oder die Arretierungsmittel (8, 18, 28) mehrere Erhebungen (9, 19, 29) und/oder Ausnehmungen (10, 20, 30) umfassen, welche hintereinander liegend angeordnet sind.

8. Vorrichtung nach nach Anspruch 6 oder 7, **dadurch gekennzeichnet, dass** das oder die Arretierungsmittel des Verstellelements korrespondierend zu dem oder den Arretierungsmittel(n) (8, 18, 28) des Befestigungsmittels (2, 12, 22) angeordnet sind.

9. Vorrichtung nach einem der Ansprüche 3 bis 7, **dadurch gekennzeichnet, dass** mindestens ein Durchmesser der zweiten Öffnung (6, 16, 26) oder der Ausnehmung des Verstellelements (5, 15, 25) reversibel veränderbar ist.

10. Vorrichtung nach einem der Ansprüche 1 bis 9, **dadurch gekennzeichnet, dass** das Befestigungsmittel (2, 12, 22) und/oder das Verstellelement (5, 15, 25) zumindest teilweise aus einem elastischen Kunststoffmaterial bestehen.

11. Vorrichtung nach einem der Ansprüche 1 bis 10, **dadurch gekennzeichnet, dass** das Befestigungsmittel (2, 12, 22) und/oder das Verstellelement (5, 15, 25) zumindest teilweise aus einem weichen Kunststoffmaterial bestehen, vorzugsweise medizinischem Silikon.
